# EUROPEAN PATENT APPLICATION

(11) **EP 4 755 323 A1**
(43) Date of publication of application: **10.06.2026**
(21) Application number: 24217146.0
(22) Date of filing: 03.12.2024
(51) Int. Cl.: A61B 17/068, A61B 17/00, A61B 17/064, A61F 2/04

(54) **DEVICE FOR ASSISTING DELIVERY AND ATTACHMENT OF A GRAFT TO A VESSEL WALL**

(71) Applicant: Universiteit Antwerpen, 2000 Antwerpen (BE); Universitair Ziekenhuis Antwerpen, 2650 Edegem (BE)
(72) Inventor: Van Campenhout, Lukas, 2020 Antwerpen (BE); Claus, Stijn, 2020 Antwerpen (BE); De Win, Gunther, 2020 Antwerpen (BE)
(74) Representative: De Clercq & Partners

(57) **Abstract**

A device (100) having a proximal end (10) and distal end (20) for delivery of a graft sheet (450) for deployment and attachment to a target within a tubular vessel (400), wherein the device (100) comprises a delivery head (200) configured for passage through the tubular vessel (400) of the subject, wherein the delivery head (200) comprises: a holding element (220) comprising a slot (222) open at least at its proximal (10) end, configured for slidable receiving of and retention of the graft sheet (450) for presentation to the target; a coupling element (260) configured for slidable attachment to a delivery tube (310) of a tacker device, wherein the tacker device is configured to deliver and deploy a tack from a terminal distal (20) end of the delivery tube (310) for attaching the graft sheet (450) to a vessel lumen interior wall (404) of the tubular vessel (400) of the subject; wherein: the coupling element (260) is configured to rotate around a single axis (262) of rotation relative to the holding element (220); the single axis (262) of rotation is disposed in fixed relation to the holding element (220); and rotational movement of the coupling element (260) around the single axis (262) of rotation to a deployment position of the coupling element (260) directs a distal end (20) of the coupling element (260) towards the slot (222).

## Description

### Field

The invention is broadly in the field of devices, in particular for repair of vessel walls in a subject.

### Background

Repair of a bodily vessel (e.g. urethra) may be performed by removal of a damaged portion, and a joining together of the lesser damaged sections. Sometimes, there is an insufficient length of vessel to remove the damaged portion, for example, in the urethra where damaged is caused by radical prostatectomy. Herein we provide a device to assist with a repair of a damaged portion of vessel, which avoids surgical sectioning and is minimally invasive.

### Summary

Provided herein is a device (100) having a proximal end (10) and distal end (20) for delivery of a graft sheet (450) for deployment and attachment to a target within a tubular vessel (400), wherein the device (100) comprises:
- a delivery head (200) configured for passage through the tubular vessel (400) of the subject, wherein the delivery head (200) comprises:
   - a holding element (220) comprising a slot (222) open at least at its proximal (10) end, configured for slidable receiving of and retention of the graft sheet (450) for presentation to the target;
   - a coupling element (260) configured for slidable attachment to a delivery tube (310) of a tacker device, wherein the tacker device is configured to deliver and deploy a tack from a terminal distal (20) end of the delivery tube (310) for attaching the graft sheet (450) to a vessel lumen interior wall (404) of the tubular vessel (400) of the subject;
wherein:
- the coupling element (260) is configured to rotate around a single axis (262) of rotation relative to the holding element (220); and
- the single axis (262) of rotation is disposed in fixed relation to the holding element (220);
- rotational movement of the coupling element (260) around the single axis (262) of rotation to a deployment position of the coupling element (260) directs a distal end (20) of the coupling element (260) towards the slot (222).

Preferably, the single axis (262) is disposed in a longitudinal position of the holding element that is within an articulation portion (290) of the delivery head (200), wherein
- the articulation portion (290) is a longitudinal portion of the holding element (220) that extends in a longitudinally proximal (10) direction from a start plane (292),
- start plane (292) that is a plane contacting a longitudinal axis (A-A') of the holding element (220) and located along the longitudinal axis (A-A') at a half of the slot (222) longitudinal length (*sl*).

Preferably, the holding element (220) comprises a pair of arms (230, 240) flanking the slot (222), wherein one arm of the pair of arms, a superior arm (230), is disposed superior (S) of the slot (222) and the single axis (262) is disposed on the superior arm (230).

Preferably, the other arm of the pair of arms, an inferior arm (240), is disposed inferior (I) of the slot (222) and is provided with an exit aperture (244), wherein the exit (244) aperture is an aperture passing between a superior (S) surface of the inferior arm (240) and an inferior (I) surface of the inferior arm (240) and which extends longitudinally to the proximal (10) end of the inferior (I) arm (240), and is open at its proximal (10) end.

Preferably, the delivery head (200) further comprises at least one retractable locking pin (270) having a deployed and retracted position, wherein
- in the deployed position at least a part a pin shaft of the at least one retractable locking pin (270) is disposed within the slot (222), and
- in the retracted position, the slot (222) is devoid of the pin shaft.

Preferably, at least one retractable locking pin (270) is biased in the deployed position.

Preferably, the least one retractable locking pin (270) is manually actuatable between the deployed position and the retracted position using a pullcord.

Preferably, the holding element (220) comprises one or more frictional contact protrusions (280) configured to increase frictional retention of the graft sheet in the slot (222).

Preferably, the delivery head (200) further comprises an atraumatic distal tip (280) disposed at a distal (20) end of the delivery head (200), and the atraumatic distal tip (280) is provided with one or more surface channels disposed in a longitudinal direction configured to provide liquid drainage over an exterior of the atraumatic distal tip (280) in a distal (20) to proximal (10) direction.

Preferably, coupling element (260) comprises a coupling element body (264) having a proximal end (10) and a distal end (20), and the coupling element body (264) comprises a through passage (266) open ended at both its proximal end (10) and its distal end (20), configured for slidable coupling with and attachment to the delivery tube (310) of the tacker (300).

Preferably, the coupling element (260) is further configured for slidable attachment to a flexible insertion tube of an endoscope, wherein the endoscope is configured to capture images from a distal tip of the flexible insertion tube.

Preferably, the coupling element body (264) further comprise a further through passage (269) configured for slidable coupling with the flexible insertion tube of the endoscope.

According to a preferred aspect:
- the rotation between the coupling element (260) and the holding element (220) around the single axis (262) is realised by a revolute (1 DOF) joint (268),
- the revolute joint (268) comprises:
   - one pair of revolute joint, RJ, first bodies (268a1, 268b1) disposed in fixed relation to the coupling element body (264), and
   - one pair of RJ second bodies (268a2, 268b2) is disposed in fixed relation to the holding element body (220, 222),
   and
- each RJ first body (268a1, 268b1) is configured to attach to the corresponding RJ second body (268a2, 268b2), thereby forming the revolute joint (268).

According to a preferred aspect:
- the slot (222) is longitudinal, and the longitudinal length of the slot is oriented in a longitudinal direction with respect to the delivery head (200);
- the axis of rotation is oriented in a lateral direction with respect to the delivery head (200).

### Brief description of the drawings

The following description of the figures of specific embodiments of the invention is merely exemplary in nature and is not intended to limit the present teachings, their application or uses.

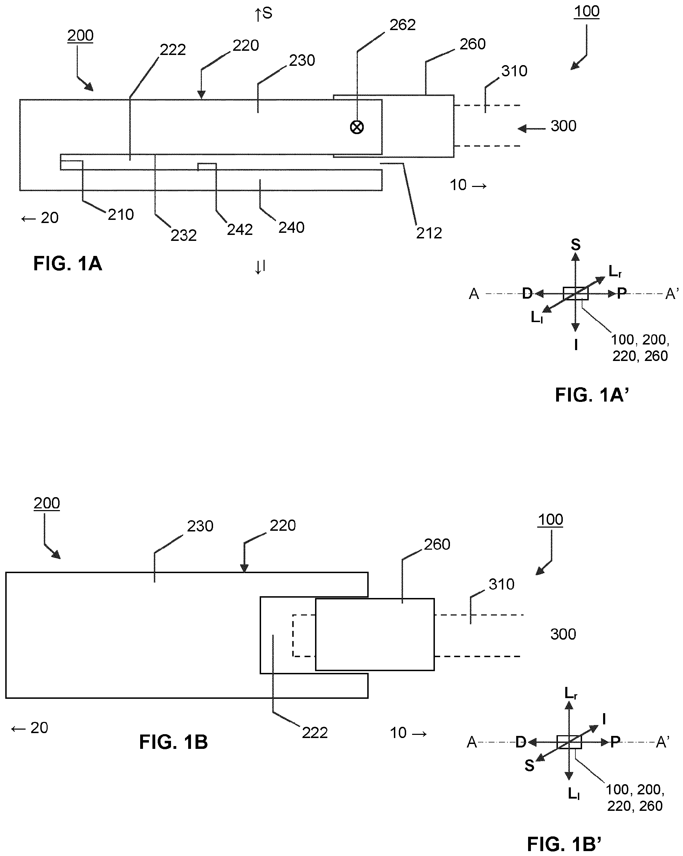

**Fig. 1A** illustrates a lateral side view of a device as described herein, where the coupling element is in a non-deployment position.
**Fig. 1A'** is an indicator of various directions of a device or delivery head or holding element or coupling element as shown in FIG. 1A.
**Fig. 1B** illustrates a superior view (superior - inferior direction) of the device of FIG. 1A.
**Fig. 1B'** is an indicator of various directions of a device or delivery head or holding element or coupling element as shown in FIG. 1B.

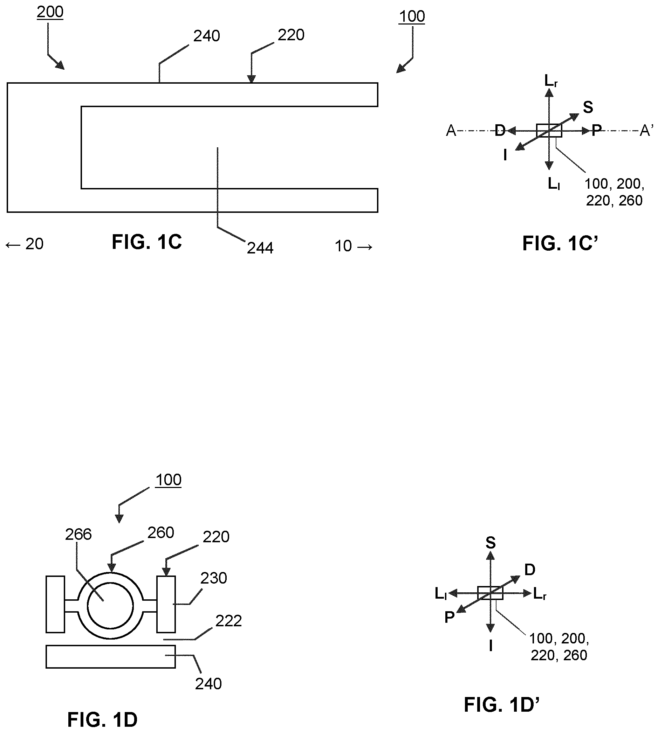

**Fig. 1C** illustrates a inferior view (inferior - superior direction) of the device of FIG. 1A.
**Fig. 1C'** is an indicator of various directions of a device or delivery head or holding element or coupling element as shown in FIG. 1C.
**Fig. 1D** illustrates a proximal-distal side view of the device of FIG. 1A.
**Fig. 1D'** is an indicator of various directions of a device or delivery head or holding element or coupling element as shown in FIG. 1D.
**Fig. 2** illustrates a lateral side view of a device as described herein, where the coupling element is in a deployment position.
**Fig. 3A** illustrates a lateral side view of a slot of a device as described herein with dimensional indicators added.
**Fig. 3B** illustrates a view along a longitudinal direction of a slot of a device as described herein with dimensional indicators added.
**Fig. 4** illustrates a lateral side view of a coupling element as described herein
**Fig. 5** illustrates a view along a longitudinal direction of a coupling element as described herein.
**Figs. 6** and **7** illustrate components of a revolute joint as described herein
**Fig. 8** illustrates a view along a longitudinal direction of a coupling element as described herein, provided with a further through passage.
**Fig. 9** illustrates the device of FIG. 1A further provided with a retractable locking pin, wherein the retractable locking pin is in the deployed position.
**Fig. 10** illustrates the device of FIG. 1A further provided with a retractable locking pin, **wherein** the retractable locking pin is in the retracted position.
**Fig. 11** illustrates the device of FIG. 1A further provided with frictional contact protrusion.
**Fig. 12** illustrates the device of FIG. 1A further provided with an atraumatic distal dip.
**Fig. 13** illustrates the device of FIG. 1A and an articulation portion, wherein the single axis of rotation is located outside the articulation portion.
**Fig. 14** illustrates the device of FIG. 1A and an articulation portion, wherein the single axis of rotation is located within the articulation portion.
**Fig. 15** illustrates the device of FIG. 1D disposed within a vessel lumen.
**Fig. 16** shows a computer aided design (CAD) view of a device described herein, wherein the device was produced using additive manufacturing.

### Description of embodiments

As used herein, the singular forms "a", "an", and "the" include both singular and plural referents unless the context clearly dictates otherwise.

The terms "comprising", "comprises" and "comprised of" as used herein are synonymous with "including", "includes", "containing", or "contains", and are inclusive or open-ended and do not exclude additional, non-recited members, elements or method steps. The terms also encompass "constituted of", "consists in", "consisting of", and "consists of', and also the terms "consisting essentially of", "consisting essentially in" and "consists essentially of', which enjoy well-established meanings in patent terminology.

The recitation of numerical ranges by endpoints includes all intervening values between the lower and upper endpoints, as well as the recited endpoints. Intervening values may be integers or, where applicable, fractions, i.e., more broadly any real numbers such as any rational numbers. This applies to numerical ranges irrespective of whether they are introduced by the expression "from... to..." or the expression "between... and..." or another expression. Any numerical range recited herein is intended to include all subranges subsumed therein. For example, each sub-range between any stated value in a stated range and any other stated value in that stated range is also specifically disclosed. Each sub-range between any stated value in a stated range and either the lower endpoint or the upper endpoint of the stated range is also specifically disclosed. The stated value may be an isolated value or an endpoint of a range subsumed by or overlapping with the stated range. For example, for a stated range with lower endpoint L1 and upper endpoint U1 (i.e., stated range L1-U1) and a stated sub-range nested within the stated range with lower endpoint L2 and upper endpoint U2 (i.e., stated sub-range L2-U2), also specifically disclosed are the subranges L1-L2, L1-U2, L2-U1, and U2-U1.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

As used herein, the term "and/or" when used in a list of two or more items, means that any one of the listed items can be employed by itself or any combination of two or more of the listed items can be employed. For example, if a list is described as comprising group A, B, and/or C, the list can comprise A alone, B alone, C alone, A and B in combination, A and C in combination, B and C in combination, or A, B, and C in combination.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art.

For example, in the appended claims, any of the claimed embodiments can be used in any combination.

Similarly, it should be appreciated that in the description of illustrative embodiments of the invention, various features of the invention are sometimes grouped together in a single embodiment, figure, or description thereof for the purpose of streamlining the disclosure and aiding in the understanding of one or more of the various inventive aspects.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and scope of the appended claims.

The terms "distal", "distally", "distal to" or "distal end" (termed "D" or "20" herein) are used throughout the specification, and are terms generally understood in the field to mean away (distal) from the user's (e.g. practitioner's) side of the device. The terms "proximal", "proximally", "proximal to" or "proximal end" (termed "P" or "10" herein) are used throughout the specification, and are terms generally understood in the field to mean towards (proximal the user's (e.g. practitioner's) side of the device.

The "subject" refers to a person receiving the graft. The "user" refers to a person or persons applying the graft. The user may be a surgeon, in particular a surgeon, clinician, physician, or medical assistant.

The term "longitudinal" is known in the art and refers to a direction or orientation in distal to proximal direction, typically along a longest dimension. The "longitudinal axis" (A-A') of the delivery head refers to a straight axis in a longitudinal direction between a distal end and a proximal end. The "longitudinal axis" of the delivery head may be "central longitudinal axis" of the delivery that is a straight axis in a longitudinal direction between a distal end and a proximal end. Typically the central longitudinal axis is disposed at a rotational centre of gravity of the delivery head.

Transverse means perpendicular to the longitudinal axis.

The superior (S) direction is a term referring to a upward (towards the sky) direction of delivery head when the delivery head is placed on a flat surface, oriented such that only one arm is in contact with the flat surface (e.g. the slot is parallel to the surface) and the single axis of rotation of the holding element (see later herein) is disposed in an upper portion of the holding element.

The inferior (I) direction is a term referring to a downward (towards the earth) direction of delivery head when the delivery head is placed on a flat surface, oriented such that only one arm is in contact with the flat surface (e.g. the slot is parallel to the surface) and the single axis of rotation of the holding element (see later herein) is disposed in an upper portion of the holding element.

The lateral (L) direction refers to a sideways (left to right, or *vice versa* from a perspective of a viewer) direction of the delivery head when the delivery head is placed on a flat surface oriented and the longitudinal axis is orientated towards the viewer.

It is understood that components described herein (e.g. delivery head, holding element, coupling element, tacker, graft sheet) may each separately have a proximal end and a distal end, and the respective ends are denoted with the same numerical indicator (e.g. proximal (P, 10), distal (D, 20)). It is understood that components described herein (e.g. delivery head, holding element, coupling element, tacker, graft sheet) may each separately have a superior (S), inferior (I), longitudinal, longitudinal axis (A-A'), and lateral (L) direction.

Directions such as proximal (P), distal (D), longitudinal axis (A-A'), lateral (Lₗ, Lᵣ), superior (S) and inferior (I) are illustrated in FIG. 1A' (referring to the device of FIG. 1A), FIG. 1B' (referring to the device of FIG. 1B), FIG. 1C' (referring to the device of FIG. 1C), and FIG. **1D**' (referring to the device of FIG. 1D).

Provided herein is a device (100) for delivery of and attachment of a graft sheet (450) to a target within a tubular vessel (400) of a subject. An exemplary device (100) is shown in **FIGs. 1A** to **1D****, 2, 9** to **16.**

The device (100) comprises a delivery head (200) configured for passage through a lumen (402) of tubular vessel (400) of a subject. The delivery head (200) comprises a holding element (220) configured for slidable receiving of and retention of the graft sheet for presentation to the target. The delivery head (200) further comprises a coupling element (260) configured for slidable attachment to a delivery tube (310) of a tacker device (300).

The tacker device (300) is configured to deliver (bring to the target) and deploy (drive into tissue) a tack from a terminal distal (20) end of the delivery tube (310) to attach the graft sheet to a (interior) wall of the tubular vessel (400) of the subject.

The coupling element (260) is configured to rotate around a single axis (262) of rotation (also known as a coupling element single rotational axis, CESRX (262)) relative to the holding element (220). The single axis (CESRX) (262) is disposed in fixed (pose - position and orientation) relation to the holding element (220).

A rotational movement of the coupling element (260) around the single axis (CESRX) (262) to a deployment position of the coupling element (260) directs a distal end (20) of the coupling element (260) towards a slot (222) of the holding element (220) configured for slidable receiving (i.e. loading) and retention of the graft sheet for presentation to the target.

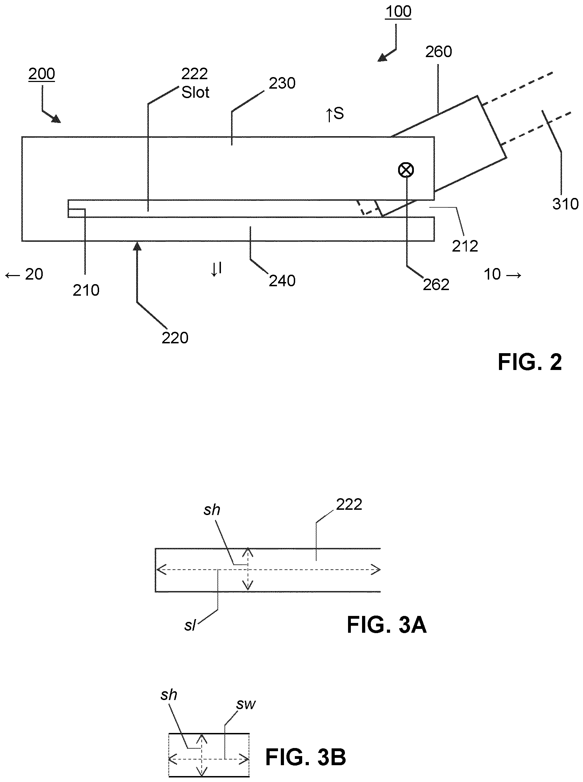

An exemplary configuration of the device (100) in the deployment position is shown in **FIGs. 2****,** **13****,** **14** and **16****.** An exemplary configuration of the device (100) in a non-deployment position is shown in **FIGs. 1A** to **1C****, 9** to **12.**

The device (100) allows an endoscopic repair of a vessel, in particular of a urethra. The provision of the rotatable coupling element (260) for coupling to the delivery tube (310) of a standard tacker, allows utilization of the standard tacker both as a fixation device and as a pusher device to advance the graft into the vessel lumen and towards the target. The provision of the rotatable coupling element (260) allows flexing of the device during passage towards the target by providing a chain-like articulation. In addition, the same rotation serves to orient the coupling element (260) to a deployment position which angles the tack in the direction of the graft sheet. The deployment position can be set externally *i.e.* by application of angular force to an exterior of the vessel, or passively due to a natural curvature of the vessel. Hence, by providing a rotatable coupling element (260), the tacker - which typically deploys the tack in a straight direction co-axial to its (inflexible) delivery tube (310) - can be brought into the direction of the graft, and at the same time, it provides flexibility needed during passage to the target.

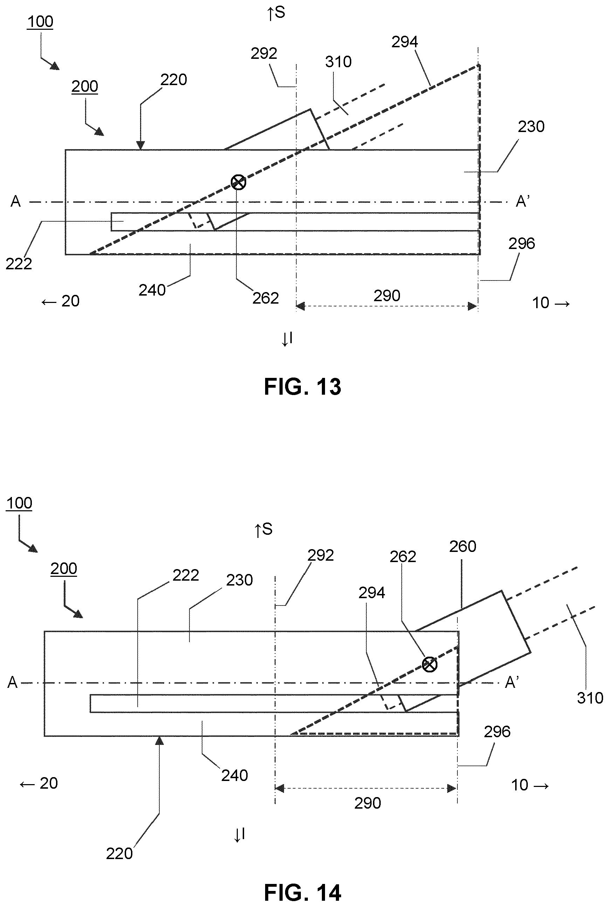

The single axis (CESRX) (262) is disposed (only or exclusively) within a longitudinal position of the holding element (220) that is within an articulation portion (290) of the holding element (220). An exemplary illustration of an articulation portion (290) is shown in FIG. 13 (the single axis (CESRX) (262) disposed within the articulation portion (290)) and in FIG. 14 (the single axis (CESRX) (262) disposed outside the articulation portion (290)). The articulation portion (290) is a longitudinal portion of holding element (220) that extends in a longitudinally proximal (10) direction from a start plane (292). The start plane (292) that is a plane contacting a longitudinal axis (A-A') of the holding element (220) and located along (contacting) the longitudinal axis (A-A') at a half of the slot (222) longitudinal length (*sl*), preferably at a third of the slot (222) longitudinal length (*sl*) (from the proximal (10) end of the slot (222)), more preferably at a quarter of the slot (222) longitudinal length (*sl*) (from the proximal (10) end of the slot (222)). The articulation portion (290) terminates at an end plane (296) that is a plane contacting the longitudinal axis (A-A') of the holding element (220) and a proximal (10) terminal end of the holding element (220).

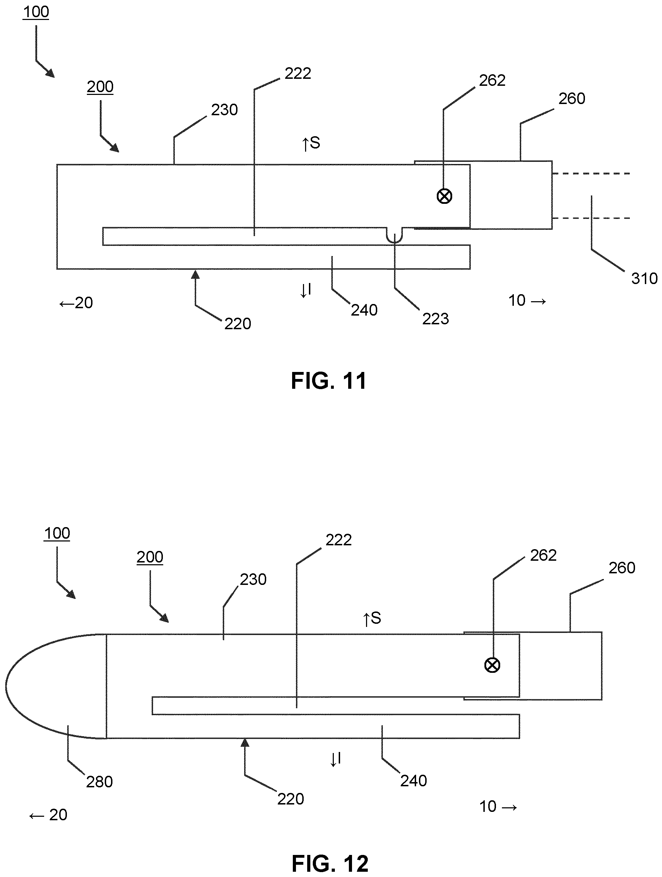

An exemplary device (100) wherein the single axis (CESRX) (262) is disposed outside the articulation portion (290) is shown in **FIG. 13** and an exemplary device (100) wherein the single axis (CESRX) (262) is disposed within the articulation portion (290) is shown in FIG. **14****.** By placing the single axis (CESRX) (262) at a longitudinal position of the delivery head (200) within the articulation portion (290), the device has a kinematic chain-like articulation wherein a rotation around the single axis (262) is accompanied by a minimal size change such that the device does not apply undue force to the lumen walls in the deployment position. If the single axis (CESRX) (262) is disposed outside the articulation portion (290), the chain formed by the delivery tube (310) of a tacker device and by the holding element (220) in the deployment position would create a large force-generating wedge (294) causing widening and unnecessary stretching of the lumen, which can lead to damage of the vessel. (size of force-generating wedge (294) in **FIG. 12** is larger cf FIG. 13). The force-generating wedge (294) is minimised when the single axis (CESRX) (262) is disposed in a longitudinal position of the device (100) that is within the articulation portion (290) having a start plane (292) disposed in the proximal quarter length of the slot (222).

The delivery head (200) comprises the holding element (220) described herein and the coupling element (260) described herein rotatably attached to the delivery head (200) at the single axis (CESRX) (262) of rotation.

The delivery head (200) is dimensioned according to the dimension of vessel lumen. Because a vessel may have some elasticity, the delivery head (200) may have a larger maximum lateral (Lₗ-Lᵣ) width the vessel could accommodate without (radial) elastic expansion. The skilled person will understand how to dimension the delivery head (200) according to the elastic expansion of the vessel. As general guidance, when the vessel is the male urethra, the delivery head (200) including any atraumatic distal tip, may have a maximum lateral (Lₗ-Lᵣ) width of 8 to 10 mm and a maximum longitudinal length of 15 mm to 50 mm.

The holding element (220) comprises a pair of arms (230, 240) flanking (both on superior (S) and inferior (I) sides of) a slot (222), wherein the slot (222) is configured for slidable receiving (i.e. loading) and retention of the graft sheet for presentation to the target. One arm (230, superior (S) arm) is disposed superior (S) of the slot (222). One arm (240, inferior (I) arm) is disposed inferior (I) of the slot (222).

The inferior arm (240) has a superior surface (242); the superior (S) surface (242) is one of a pair of graft sheet (GS) contact surfaces. The superior arm (230) has an inferior (I) surface (232); the inferior surface (232) is the other of the pair of GS contact surfaces. The one or both GS contact surfaces may be smooth, roughened, disposed with one or more frictional contract protrusions (described elsewhere herein).

Preferably the superior (S) arm (240) is disposed with the single axis (CESRX) (262). Preferably, the single axis (CESRX) (262) is attached to or placed on the superior (S) arm (e.g. 240).

The inferior (I) arm (240) is provided with an exit aperture (244) through which the deployed tack exits the delivery head (200) after passing through the sheet graft (150). The exit aperture (244) (see e.g. **FIG. 1C**) is an aperture in the inferior (I) arm (240) passing between a superior surface (242) of the inferior (I) arm (240) and an inferior surface of the inferior (I) arm (240) and which extends longitudinally to the proximal (10) end of the inferior (I) arm (240). The exit aperture (244) is open at its proximal (10) end. The exit aperture (244) is configured for slidable passage of the tack attached to the vessel wall when the device (100) is advanced through the vessel. The exit aperture preferably has a rectangular shape e.g. oblong.

The placement of the single axis (CESRX) (262) on the superior arm (e.g. 230) allows the coupling element to be directed in an inferior (I) direction and towards the graft sheet and vessel wall. The placement of the exit aperture (242) on the inferior arm (e.g. 240) allows a placement of multiple tacks in a graft sheet, since a previously deployed tack attached to the graft sheet and vessel wall can exit the delivery head (200) *via* the exit aperture (242), and the delivery head (200) can be repositioned, by further advancing into the vessel, for deployment of a further tack from the tacker.

Each arm (230,240) is longitudinal. The arms (230,240) may have same lengths or different lengths. A length of an arm is a longitudinal distance from the end stop (210) to a proximal terminal end of the arm. Where the arms (230,240) have the different lengths, the slot maximum length is defined by the shorter of two arms (230,240).

As mentioned, the holding element (220) comprises the pair of arms (230, 240) flanking a slot (222) configured for slidable receiving (i.e. loading) and retention of the graft sheet for presentation to the target. The slot (222) is disposed between the pair of arms (230,240); The slot has a proximal end, an distal end, and lateral sides.

As a general guidance a slot (222) for holding a graft sheet for repair of a urethra wall:
- may have a point of maximum height (sh) that is 2 mm to 4 mm; and/or
- may have a point of maximum width (sw) of 8 mm to 11 mm; and/or
- may have a longitudinal point of maximum length (*sl*) of 20 mm to 40 mm.

Dimensional indications are shown in **FIG. 3A** and 3B. It is appreciated that the slot (222) dimension may be adjusted according to a dimension of the lumen.

A form that is a rectangular prism may fit within the slot. A form that is wedge-shaped prism may fit within the slot. A wider end of the slot may be disposed at a longitudinal distal end of the slot. Alternatively, a wider end of the slot may be disposed at a longitudinal proximal end of the slot. Measurements and/or forms are considered in the absence of the coupling element (260), and of any frictional contact protrusions (280) that protrude into the slot.

The slot (222) has an end stop (210) disposed at the distal end of the slot (222) configured for abutting with an edge of the sheet graft. The end stop (210) is typically formed by a connecting piece between the pair of arms (230,240).

The slot has an open proximal edge (212) disposed at the proximal end of the slot (222) configured for slidable unloading of the graft sheet (after deployment of the graft sheet, by advancement of the delivery head in a distal direction). By providing an open proximal edge (212) at the proximal end of the slot (222), multiple tacks can be applied to the graft sheet spaced apart during the slidable unloading of graft sheet.

The slot may have open lateral (Lₗ, Lᵣ) edge(s) disposed at a lateral (Lₗ, Lᵣ) sides of the slot (222) configured to allow a graft sheet larger in width than the slot to extend outside the slot.

The holding element (220) may be made from any suitable biocompatible material. Examples of suitable materials include a medical-grade polymer (e.g. polytetrafluoroethylene (PTFE), polyethylene, polycarbonate) or a medical-grade metal (e.g. Titanium or stainless steel).

The holding element (220) may be a monoblock *i.e.* a single piece. The single piece is made from the same material. The single piece may be made by any suitable process such as additive manufacturing, injection moulding, milling, and the like.

The holding element (220) may be a multi-element *i.e.* a combination of multiple elements that co-operate to form the coupling element (260). Each of the multiple elements may be made by any suitable process such as additive manufacturing, injection moulding, milling, and the like. The multiple elements may be connected using any suitable process e.g. adhesive, interference fitting, and the like. Each of the multiple elements piece may be made from the same material. At least two of the multiple elements piece may be made from different materials.

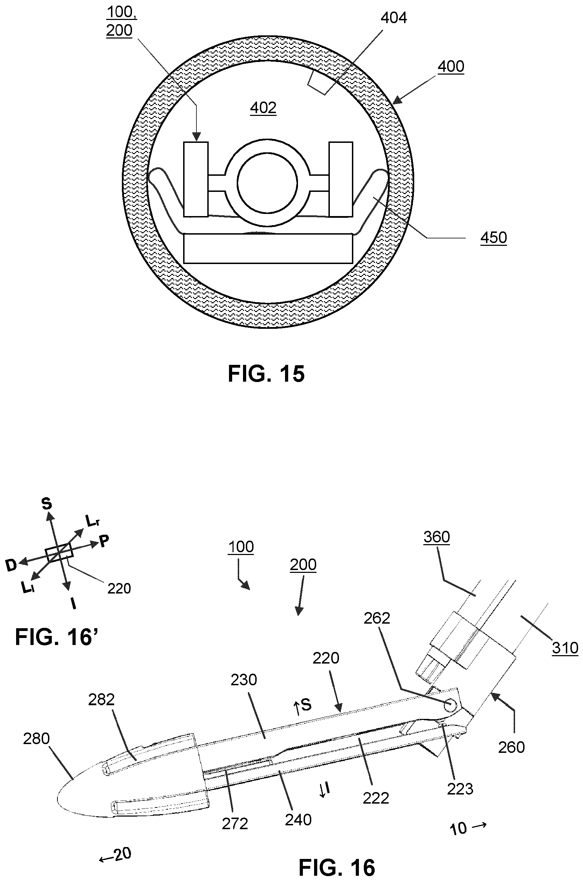

The tubular vessel (400) may be any bodily tubular vessel. A tubular vessel (400) typically has a vessel lumen (402) and a vessel lumen interior wall (404). The tubular vessel may terminate in an exterior natural orifice. The tubular vessel may have an orifice (entry point for the delivery head) that is an incision. Examples of tubular vessels (400) include urethra, male urethra, oesophagus, small intestine, and rectal passage. An exemplary bodily vessel (400) is shown in FIG. 15, with device (200) described herein disposed in the vessel lumen (402).

The target of bodily tubular vessel is typically a portion of the vessel lumen interior wall (404) in need of the graft sheet. In particular, the target may be a damaged portion of the vessel lumen interior wall (404).

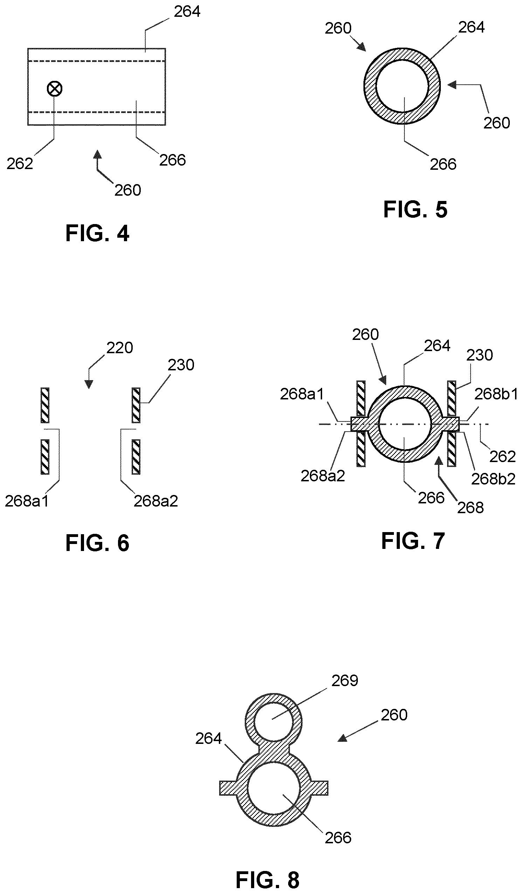

The coupling element (260) comprises a coupling element body (264) having a proximal end (10) and a distal end (20). An exemplary coupling element (260) is shown in detail in FIGs. 4, 5, 7, and 8. The coupling element body (264) comprises a through passage (266) configured for slidable coupling with and attachment to the delivery tube (310) of the tacker (300).

The through passage (266) is open ended at both proximal end (10) and distal end (20). Through passage (266) is preferably cylindrical in shape (circular transverse profile). A central axis of the through passage (266) is preferably disposed parallel to a plane perpendicular to the single axis (CESRX) (262). The through passage (266) is configured for slidable coupling with a distal end portion of the delivery tube. Rotational movement of the coupling element (260) around the single axis (CESRX) (262) to the deployment position of the coupling element (260) directs a distal end (20) of the through passage (266) towards the slot (222).

A pose (position and orientation) of the distal end portion of the delivery tube (310) with respect to the coupling element body (264) may be adjustable or non-adjustable. Once a desired pose has been reached by the user, the desired pose is fixed or fixable. By fixed or fixable, it is meant that the pose of the distal end portion of the delivery tube (310) relative to the coupling element body (264) does not change at least during the deployment of the graft sheet to the target and the attachment of the graft sheet to the target within a tubular vessel of a subject. The pose that is fixed or fixable may be achieved by any means configured to fix the pose, such as an interference fit, a form-fit, one or more interlocking elements (bayonet fit), or one or more clamps.

For example, the through passage (266) may be configured for slidable coupling with a distal end portion of the delivery tube such that a pose (position and orientation) of the distal end portion of the delivery tube (310) with respect to the coupling element body (264) is fixed or fixable by a clamping element (e.g. threaded rod) that is actuatable (e.g. by an axial rotation of the rod).

For example, the through passage (266) may be configured for slidable coupling with a distal end portion of the delivery tube (310) such that a pose (position and orientation) of the distal end portion of the delivery tube (310) with respect to the coupling element body (264) is fixed by an interference fit between the (interior wall of the) through passage (266) and the (exterior wall of) the distal end portion of the delivery tube (310). In particular, an axial position of the coupling element (260) relative to the delivery tube (310) may be fixed by an interference fit between the (interior wall of the) through passage (266) and the (exterior wall of) the distal end portion of the delivery tube (310). In particular, an axial rotation of the coupling element (260) relative to the delivery tube (310) may be fixed by an interference fit between the (interior wall of the) through passage (266) and the (exterior wall of) the distal end portion of the delivery tube (310). The pose (position and orientation) of the distal end portion of the delivery tube (310) with respect to the coupling element body (264) is fixed after a desired pose of the distal end portion of the delivery tube (310) with respect to the coupling element body (264) has been met by application of a force (pushing and/or rotational) to the delivery tube (310).

The coupling element (260) is configured to rotate around (only) a single axis (CESRX) (262) relative to the holding element (220). The coupling element (260) is rotatably attached to holding element (220), where the rotatable attachment is around (only) the single axis (CESRX) (262) (only 1 degree of freedom of rotation) relative to the holding element (220).

The rotation of the coupling element (260) relative to the holding element (220) (e.g. in a first direction) may bring the coupling element (260) into the deployment position. In the deployment position, the coupling element (260) directs a distal end (20) of the coupling element (260) (in particular a distal end (20) of the through passage (266)) towards the slot (222) of the holding element (220) such that the tacker is able to direct the tack into the graft retained by the slot (222). An example of a deployment position is shown in **FIGs. 2****,** 14 and 16.

The rotation of the coupling element (260) relative to the holding element (220) (e.g. in a second direction opposite to the first direction) may bring the coupling element (260) into a non-deployment position. In the non-deployment position, the coupling element (260) directs a distal end (20) of the coupling element (260) (in particular a distal end (20) of the through passage (266)) away from the slot (222) of the holding element (220) such that a tack from the tacker is not directed into the graft retained by the slot (222). An example of a non-deployment position is shown in **FIGs. 1A to 1B****, 9** to **12.**

A range of rotational movement of the coupling element (260) may be at least 30 deg, preferably less than 100 deg.

The single axis (CESRX) (262) is disposed in fixed (pose - position and orientation) relation to the holding element (220), preferably in fixed relation to the superior arm (240).

The single axis (CESRX) (262) is preferably disposed superior (S) of the holding element slot (222). The single axis of rotation (CESRX) (262) is preferably disposed perpendicular to a longitudinal axis of the delivery head (200). The single axis (CESRX) (262) is preferably disposed in a lateral (Lᵣ-Lₗ) direction.

The single axis (CESRX) (262) preferably contacts a centre of gravity of a transverse cross section of the coupling element through passage (266). The centre of gravity is a centre of a circle when a transverse cross section of the coupling element through passage (266) is circular.

The coupling element (260) may be made from any suitable biocompatible material. Examples of suitable materials include a medical-grade polymer (e.g. polytetrafluoroethylene (PTFE), polyethylene, polycarbonate) or a medical-grade metal (e.g. titanium or stainless steel).

The coupling element (260) may be a monoblock *i.e.* a single piece. The single piece is made from the same material. The single piece may be made by any suitable process such as additive manufacturing, injection moulding, milling, and the like.

The coupling element (260) may be a multi-element *i.e.* a combination of multiple elements that co-operate to form the coupling element (260). Each of the multiple elements may be made by any suitable process such as additive manufacturing, injection moulding, milling, and the like. The multiple elements may be connected using any suitable process e.g. adhesive, interference fitting, and the like. Each of the multiple elements piece may be made from the same material. At least two of the multiple elements piece may be made from different materials.

The rotation between the coupling element (260) and the holding element (220) around the single axis (CESRX) (262) is preferably realised by a revolute joint (268) (also known as an "articulating revolute joint"). A revolute joint is understood in the art to have a single (only one) degree of freedom of rotation. The revolute joint (268) and its elements are exemplarity shown in **FIGs. 6** and **7****.** The revolute joint both attaches the coupling element (260) and the holding element (220) together, and limits rotation between the coupling element (260) and the holding element (220) around the single axis (CESRX) (262).

The revolute joint preferably comprises:
- one pair of revolute joint (RJ) first bodies (268a1, 268b1) disposed in fixed relation to the coupling element body (264), and
- one pair of revolute joint (RJ) second bodies (268a2, 268b2) is disposed in fixed relation to the holding element body (220, 222).

Each RJ first body (268a1, 268b1) is configured to attach to the corresponding second hinge body (268a2, 268b2).

Each RJ first body (268a1, 268b1) is configured to attach to each corresponding RJ second body (268a2, 268b2) such that the coupling element body (264) is able to rotate relative to the holding element body (220, 222) around (only) the single axis (CESRX) (262).

Each RJ first body (268a1, 268b1) preferably comprises a lateral protrusion. Each RJ second body (268a2, 268b2) preferably comprises a complementary hole that receives the RJ first body (268a1, 268b1) lateral protrusion.

The pair of RJ first bodies (268a1, 268b1) is arranged disposed in fixed relation to the coupling element body (264) such that they allow rotation of the coupling element body (264) around the single axis (CESRX) (262) when the pair of RJ second bodies (268a2, 268b2) are revolutely attached to the pair of RJ first bodies (268a1, 268b1).

A one pair of RJ second bodies (268a2, 268b2) is disposed in fixed relation to the holding element body (220, 222), more in particular in fixed relation to one or both of the arms (230, 240, preferably in fixed relation to the superior arm (230).

Central axes of each RJ first body (268a1, 268b1) are mutually co-axial. Central axes of each RJ second body (268a2, 268b2) are mutually co-axial.

A tacker device is a device known in the art for fixation of a graft sheet to soft tissue using a tack delivered by the device. The delivery tube (310) is preferably from a standard (off-the-shelf) tacker device (300). Examples of suitable off-the-shelf tacker devices (300) include those manufactured by Ethicon (e.g. Securestrap), Covidien/Medtronic (e.g. Protack).

The delivery tube (310) typically has a standardised shape. It allows the shape and size of the through passage (266) to be determined and/or optimised in order fix the pose (position and orientation) of the distal end portion of the delivery tube (310) with respect to the coupling element body (264), for instance, by the interference fit between the (interior wall of the) through passage (266) and the (exterior wall of) the distal end portion of the delivery tube (310).

The graft sheet (450) may be any type of sheet suitable for attachment to a wall of a vessel. The graft sheet may be a buccal mucosa or an artificial graft. The graft sheet has a sheet-like form. The graft sheet may have an essentially uniform thickness across the sheet (e.g. have a rectangular prism form). The graft sheet may have a variable thickness across the sheet. For instance, the graft sheet may have a gradually increasing or decreasing thickness in a longitudinal direction (*e.g*. have a wedge-shaped prism form). The graft sheet may be disposed with one or more shapes configured to assist in holding by the holding element e.g. one or more apertures, one more lateral side arms, one or more (superior and/or inferior) protrusions. When held in the holding element, it typically conforms to a shape of the slot (222); any overhanging portions of the graft sheet (450) typically conform to the shape of the lumen inner wall during delivery to the target.

As a general guidance for attachment to an interior wall of a urethra of a subject, the graft sheet may have a maximum thickness of 0.5mm to 2 mm, a maximum width of 11 mm to 15 mm and a maximum length of 30 mm to 40 mm.

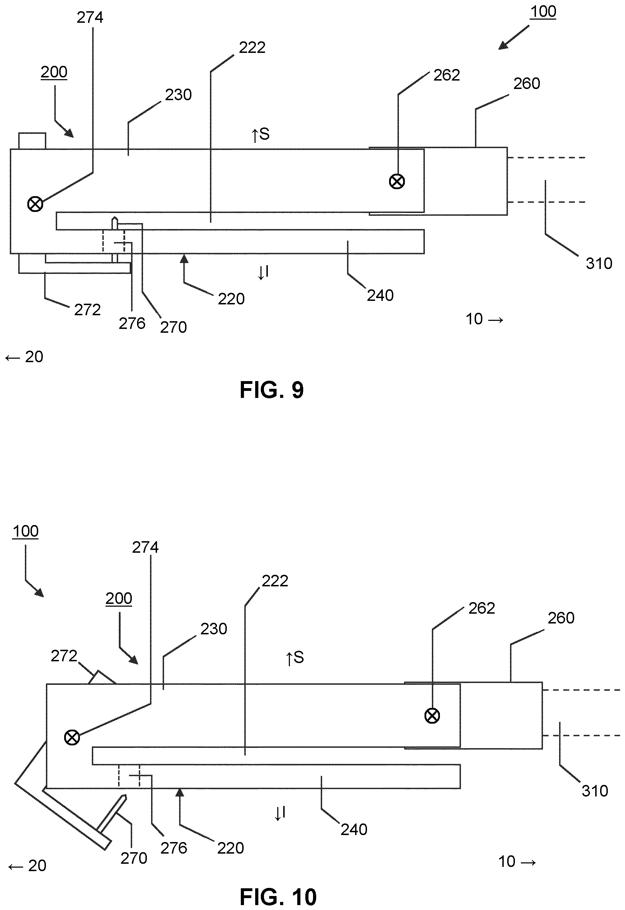

The holding element (220) may further comprise at least one retractable locking pin (270) having a deployed and retracted position. An exemplary configuration of the holding element (220) retractable locking pin (270) is shown in **FIGs. 9** and **10****.** The retractable locking pin has a pin shaft. One end of the pin shaft (a leading end of the shaft) is configured to pierce the graft sheet. The leading end of the shaft end may or may not be pointed. The retractable locking pin is associated (e.g. by attachment) with a **mechanism** (e.g. lever or lever mechanism described below) for movement of the pin shaft between deployed and retracted positions.

In the deployed position (*e*.*g*. **FIG. 9**) at least a part of the pin shaft is disposed within the slot (222) of the holding element (220). Preferably, in the deployed position, the least a part of the pin shaft is disposed within the slot (222) of the holding element (220) oriented in a (net) inferior to superior direction. Preferably, in the deployed position, the least a part pin shaft extends across a width of the slot (222). In the deployed position, the retractable locking pin(s) (270) is configured to pierce the graft sheet thereby locking the position of the graft sheet relative to the holding element (220). Rotation of the graft may be reduced by the presence of the stop member (210) and/or when a plurality of spaced apart retractable locking pins (270) is provided.

In the retracted position (*e*.*g*. FIG. 10) the slot (222) is devoid of the pin shaft of the retractable locking pin(s) (270). In the retracted position the graft sheet is releasable from the slot (222).

The at least one retractable locking pin (270) may pass through a pin aperture (276) provided in the inferior (I) arm (240) in the deployed position. The pin aperture is an aperture in the inferior (I) arm (240) passing between a superior surface (242) of the inferior (I) arm (240) and an inferior surface of the inferior (I) arm (240).

The retractable locking pin(s) is preferably disposed biased in the deployed position. By biased, it is meant that an application of an external force moves the retractable locking pin(s) from the deployed position towards the retracted position, and after release of the external force the retractable locking pin(s) returns to the biased deployed position. The biased position may be maintained using a spring (e.g. torsion spring).

Movement of the retractable locking pin(s) between the deployed position and retracted position is manually actuatable. The retractable locking pin(s) may be manually actuated by a pullcord. Application of a pulling force preferably moves the retractable locking pin(s) from the deployed position to the retracted position. Release of the pulling force or an application of a pulling force preferably moves the retractable locking pin(s) from the retracted position to the deployed position.

A path of the pullcord is preferably radially close to a central longitudinal axis of the delivery head (200). According to one aspect, a path of the pullcord contacts the single axis (CESRX (262)) (e.g. contacted lateral sides of the single axis (CESRX (262)). According to one aspect, the pullcord is provided as a Bowden cable (e.g. the pull cord is an inner cable relative to a hollow outer cable housing, and a distal end of the hollow outer cable housing is disposed in essentially fixed relation to the holding element (220)).

The retractable locking pin(s) may be disposed at a first end of a lever (272). A second other end of the lever, opposing the first end of a lever (272) may be attached to the pullcord. Application of tension to the pullcord causes movement of the retractable locking pin(s) towards the retracted position. The lever is a part of a lever mechanism for movement of the pin shaft between deployed and retracted positions. The lever mechanism may further include a lever revolute joint at a lever fulcrum (274), and a torsion spring to maintain the lever and hence retractable locking pin(s) biased in the deployed position. The lever revolute joint allows rotation of the lever (272) relative to the holding element (220), in particular relative to the slot (222). The lever (272) may be any suitable shape, for instance, to complement a shape of the delivery head (200) or holding element (220). The lever mechanism may be configured such that the retractable locking pin(s) passes through the pin aperture provided in the inferior (I) arm (240) during movement to the deployment position and/or during movement to the retracted position.

Quantity of retractable locking pin(s) or pin shafts may be 1, 2 or more. The one or plurality of retractable locking pin(s) or pin shafts is/are provided at the first end of the lever (272).

The retractable locking pin(s) in the deployed position is able to penetrate the graft, thereby locking the position (i.e. displacement) of the graft; this increases secure fixation of the graft to the delivery head (200) during passage to the target. An ability to retract the retractable locking pin(s) *in situ* upon manual actuation allows deployment of the graft sheet selectively at the target. Manual actuation by the pull cord while the delivery head is *in situ* allows a possibility for manual actuation outside the urethra, increasing simplicity and usability of the device. Simplicity is important because of the restricted space (transverse cross-sectional space) within the vessel such as the urethra. Manual actuation by the pull cord allow any standard (off-the-shelf) tacker and optionally endoscope to be used, as these do not need to be adapted to include an actuation mechanism nor an additional lumen to retain a connection to the delivery head (200). Utilising the end stop (210) in combination with the retractable locking pin(s) (270) prevents or reduces both displacement and rotation of the sheet graft retained by the holding element (220) slot (222). By maintaining the retractable locking pin(s) biased in the deployed position, its passage to the target does not require application of any external force (e.g. from a push cable), thereby further simplifying design.

The retractable locking pin(s) in particular the pin shaft may be made from any suitable biocompatible material. Examples of suitable materials include a medical-grade polymer (e.g. polytetrafluoroethylene (PTFE), polyethylene, polycarbonate) or a medical-grade metal (e.g. Titanium or stainless steel).

The lever (272) may be a monoblock *i.e.* a single piece. The single piece is made from the same material. The single piece may be made by any suitable process such as additive manufacturing, injection moulding, milling, and the like.

The lever (272) may be a multi-element *i.e.* a combination of multiple elements that co-operate to form the lever (272). Each of the multiple elements may be made by any suitable process such as additive manufacturing, injection moulding, milling, and the like. The multiple elements may be connected using any suitable process e.g. adhesive, interference fitting, and the like. Each of the multiple elements piece may be made from the same material. At least two of the multiple elements piece may be made from different materials.

The holding element (220) may further comprise one or more frictional contact protrusions (223). The one or more frictional contact protrusions (223) is configured to increase frictional retention of the graft sheet in the holding element (220), in particular in the slot (222). The one or more frictional contact protrusions (223) is provided on one or both arms (230, 240) and projects into the slot (222). A local width of the slot at a position of the frictional contact protrusion (223) is reduced; the reduction in local slot width is caused by the presence of the frictional contact protrusion (223). The one or more frictional contact protrusions (223) is configured to increase friction between the graft sheet and the arms (230, 240). The one or more frictional contact protrusions (223) is configured to pinch graft sheet between the arms (230, 240). The one or more frictional contact protrusions (223) is configured to reduce displacement and/or rotation of the graft relative to the holding element (220), in particular the arms (230, 240) of the holding element (220). A frictional contact protrusion (223) may have a bullet shaped or rounded tip.

At least two frictional contact protrusions (223) may be provided, wherein:
- at least one frictional contact protrusions (223) is provided on each arm, or
- at least two frictional contact protrusions (223) are provided on one arm.

Where one or more frictional contact protrusions (223) is provided on the superior arm **(230),** they are provided on an inferior surface (232) of the superior arm (230). Where one or more frictional contact protrusions (223) is provided on the inferior arm (240), they are provided on a superior surface (242) of the inferior arm (240). At least two frictional contact protrusions (223) may be provided disposed towards a proximal end of the slot (222).

The delivery head (200) may further comprise an atraumatic distal tip (280) disposed at a distal (20) end of the delivery head (200). An atraumatic tip is a tip that is not sharpened to an extent that would cause soft-tissue injury, for instance, by cutting or abrasing. The atraumatic distal tip (280) has a rounded distal terminal end e.g. bullet shaped, dome shaped. A transverse cross section of the atraumatic distal tip (280) preferably has a rounded form, e.g. circular or oval (prior to addition of channels). A maximum width of transverse cross section may be 6 mm to 10 mm.

The atraumatic distal tip (280) may be provided with one or more surface channels disposed in a longitudinal direction. A channel is a longitudinal recess in a surface of the atraumatic distal tip (280). The one or more surface channels are configured to provide liquid drainage over an exterior of the atraumatic distal tip (280) in a distal (20) to proximal (10) direction.

The atraumatic distal tip (280) may be made from any suitable biocompatible material. Examples of suitable materials include a medical-grade polymer (e.g. polytetrafluoroethylene (PTFE), polyethylene, polycarbonate) or a medical-grade metal (e.g. Titanium or stainless steel).

The atraumatic tip (280) and a holding element (220) may be formed as a one-piece unit (e.g. by injection moulding). The atraumatic distal tip (280) may be a monoblock *i.e.* a single piece with the holding element (220). The single piece is made from the same material. The single piece may be made by any suitable process such as additive manufacturing, injection moulding, milling, and the like.

The atraumatic distal tip (280) and remainder of the holding element (220) may be multi-element *i.e.* a combination of multiple elements that co-operate to form the holding element (220) with atraumatic distal tip (280). Each of the multiple elements may be made by any suitable process such as additive manufacturing, injection moulding, milling, and the like. The multiple elements may be connected using any suitable process e.g. adhesive, interference fitting, and the like. Each of the multiple elements piece may be made from the same material. At least two of the multiple elements piece may be made from different materials.

The atraumatic distal tip (280) helps to widen the vessel lumen during advancement of the delivery head (200) through the vessel lumen. The one or more surface channels where present provide liquid drainage in a distal to proximal direction. Since the space distal of the delivery head (200) is continuously irrigated, the one or more surface channels prevent a build-up of pressure distal (20) of the delivery head (200).

The coupling element (260) may be further configured for slidable attachment to a flexible insertion tube (FIT) of an endoscope, wherein the endoscope is configured to capture images from a distal tip of the flexible insertion tube (FIT).

In particular, the coupling element body (264) may further comprise a further through passage (269) configured for slidable coupling with the flexible insertion tube (FIT) of the endoscope. An exemplary further through passage is shown in FIG. 8. The further through passage (269) is open ended at both its proximal end (10) and its distal end (20).

A central axis of the coupling element further through passage (269) is disposed parallel to a plane perpendicular to the single axis (CESRX) (262).

A central axis of the coupling element further through passage (269) is disposed parallel to central axis of the coupling element through passage (266).

The further through passage (269) is configured for slidable coupling with a distal end portion of the delivery tube.

A pose (position and orientation) of the distal end portion of the flexible insertion tube (FIT) with respect to the coupling element body (264) may be adjustable or non-adjustable. Once a desired pose has been reached by the user, the desired pose is fixed or fixable. By fixed or fixable, it is meant that the pose of the distal end portion of the flexible insertion tube (FIT) relative to the coupling element body (264) does not change at least during the deployment of the graft sheet to the target and the attachment of the graft sheet to the target within a tubular vessel of a subject. The pose is fixed or fixable may be achieved by any means configured to fixed the pose, such as an interference fit, a form-fit, one or more interlocking elements, or one or more clamps.

For example, the through passage (266) may be configured for slidable coupling with a distal end portion of the flexible insertion tube (FIT) such that a pose (position and orientation) of the distal end portion of the flexible insertion tube (FIT) with respect to the coupling element body (264) is fixed by a clamping element (e.g. threaded rod) that is actuatable (e.g. by an axial rotation of the rod).

For example, the further through passage (269) may be configured for slidable coupling with a distal end portion of the flexible insertion tube (FIT) such that a pose (position and orientation) of the distal end portion of the flexible insertion tube (FIT) with respect to the coupling element body (264) is fixed by an interference fit between the (interior wall of the) through passage (266) and the (exterior wall of) the distal end portion of the flexible insertion tube (FIT). In particular, an axial position of the coupling element (260) relative to the flexible insertion tube (FIT) may be fixed by an interference fit between the (interior wall of the) through further through passage (269) and the (exterior wall of) the distal end portion of the flexible insertion tube (FIT). In particular, an axial rotation of the coupling element (260) relative to the flexible insertion tube (FIT) may be fixed by an interference fit between the (interior wall of the) further through passage (269) and the (exterior wall of) the distal end portion of the flexible insertion tube (FIT). The pose (position and orientation) of the distal end portion of the flexible insertion tube (FIT) with respect to the coupling element body (264) is fixed after a desired pose of the distal end portion of the flexible insertion tube (FIT) with respect to the coupling element body (264) has been met by application of a force (pushing and/or rotational) to the flexible insertion tube (FIT).

The flexible insertion tube (FIT) typically has a standardised shape. It allows the shape and size of the further through passage (269) to be determined and/or optimised in order to fix the pose (position and orientation) of the distal end portion of the flexible insertion tube (FIT) with respect to the coupling element body (264), for instance, by the interference fit between the (interior wall of the) further through passage (269) and the (exterior wall of) the distal end portion of the delivery tube (310).

Once the target has been located by the endoscope disposed adjacent to the target, the delivery head (200) can be axially rotated by a combined axial rotation of the tacker delivery tube (310) and flexible insertion tube (FIT) of the endoscope.

The coupling element (260) allows the axial position of the distal terminal tip of
- the delivery tube (310) of the tacker, and
- the flexible insertion tube of the endoscope
to be set independently.

Thus, the position of:
- the distal terminal tip of the delivery tube (310) of the tacker can be set to position the tack-to-be deployed at a desired position with respect to the graft sheet, and
- the distal terminal tip of the flexible insertion tube of the endoscope can be positioned axially proximal of the terminal tip of the delivery tube (310), so as to observe the deployment of the tack by the endoscope.

The provision for a coupling to a flexible insertion tube of an endoscope allows the operator of the device (100) to locate the target. The provision of a slidable coupling allows position of the distal terminal tip of the flexible insertion tube of the endoscope to be adjustable and axially offset from the distal terminal tip of the delivery tube (310) of the tacker, allowing the distal terminal tip of the delivery tube (310) to be observed endoscopically.

The device (100) may further comprise the tacker (300). The device (100) may further comprise the flexible insertion tube of the endoscope and optionally the endoscope.

Further provided is a method for delivery of and attachment of a graft sheet (450) to a target within a tubular vessel (400) of a subject, comprising:
- providing a device (100) as described herein, wherein the delivery head (200) coupling element (260) is slidably attached to a delivery tube (310) of a tacker device (300), and the graft sheet disposed within the slot (222);
- inserting the delivery head (200) into a lumen (402) of the tubular vessel (400), and advancing (in a distal direction) the delivery head (200) along the lumen (402) to the target by application of pushing force to the delivery tube (310) of the tacker device (300);
- rotating the coupling element (260) (around its revolute joint (268)) into the deployment position;
- deploying a tack from the delivery tube (310) of the tacker device (300) into the graft sheet and into a wall of the tubular vessel (400);
- optionally advancing the delivery head (200) (in a distal direction) and deploying one or more additional tacks from the delivery tube (310) of the tacker device (300) into the graft sheet and into a wall of the tubular vessel (400);
- advancing the delivery head (200) (in a distal direction) until the graft sheet is unloaded from the slot (222);
- withdrawing the delivery head (200) (in a proximal direction) from the lumen (402) by application of a pulling force to the delivery tube (310) of the tacker device (300).

### Examples

A three-dimensional CAD drawing of a working device (100) as described herein that was prepared by additive manufacturing (3D printing), and tested in a vessel mimetic is shown in **FIG. 16****.** The device (100) had a proximal end (10) and distal end (20) and comprised a delivery head (200) comprising a holding element (220) and a coupling element (260). The holding element (220) comprised a slot (222) open on its lateral (Lₗ-Lᵣ) sides and on its proximal (10) end, and the slot (222) is flanked by a superior arm (220) and an inferior arm (240). A frictional contact protrusion (280) is indicated; two frictional contact protrusions (280) (1 per arm shown) were provided on each arm (230, 240) projecting into the slot (222). The delivery head (200) further comprised an atraumatic tip (280) having a plurality of surface channels (282). A part of a lever (272) of a lever mechanism for a retractable locking pin is shown.

The coupling element (260) was revolutely attached to the superior arm (230) and rotated around the single axis (CESRX) (262) relative to the holding element (220). The coupling element (260) was attached to a delivery tube (310) of a tacker device and to a flexible insertion tube (FIT) (360) of an endoscope. The coupling element (260) is shown rotated around the around the single axis (CESRX) (262) to a deployment position in which the tacker was able to direct the tack deployed from the delivery tube (310) into the graft retained by the slot (222).

The working device (100) was prepared by 3D printing, was tested in a vessel mimetic, and was able to be advanced along the vessel mimetic under a pushing force from the delivery tube (310) of a tacker device (300), the deployment position of the coupling element (260) was able to be set, and a plurality of tacks from the tacker device (300) was able to be deployed through a graft mimetic and into the vessel mimetic, thereby securing the graft mimetic to the vessel mimetic.

## Claims

1. A device (100) having a proximal end (10) and distal end (20) for delivery of a graft sheet (450) for deployment and attachment to a target within a tubular vessel (400), wherein the device (100) comprises:
- a delivery head (200) configured for passage through the tubular vessel (400) of the subject, wherein the delivery head (200) comprises:
- a holding element (220) comprising a slot (222) open at least at its proximal (10) end, configured for slidable receiving of and retention of the graft sheet (450) for presentation to the target;
- a coupling element (260) configured for slidable attachment to a delivery tube (310) of a tacker device, wherein the tacker device is configured to deliver and deploy a tack from a terminal distal (20) end of the delivery tube (310) for attaching the graft sheet (450) to a vessel lumen interior wall (404) of the tubular vessel (400) of the subject;
wherein:
- the coupling element (260) is configured to rotate around a single axis (262) of rotation relative to the holding element (220);
- the single axis (262) of rotation is disposed in fixed relation to the holding element (220);
- rotational movement of the coupling element (260) around the single axis (262) of rotation to a deployment position of the coupling element (260) directs a distal end (20) of the coupling element (260) towards the slot (222).

2. The device (100) according to claim 1, wherein the single axis (262) of rotation is disposed in a longitudinal position of the holding element that is within an articulation portion (290) of the delivery head (200), wherein
- the articulation portion (290) is a longitudinal portion of the holding element (220) that extends in a longitudinally proximal (10) direction from a start plane (292),
- start plane (292) that is a plane contacting a longitudinal axis (A-A') of the holding element (220) and located along the longitudinal axis (A-A') at a half of the slot (222) longitudinal length (*sl*).

3. The device (100) according to claim 1 or 2, wherein the holding element (220) comprises a pair of arms (230, 240) flanking the slot (222), wherein one arm of the pair of arms, a superior arm (230), is disposed superior (S) of the slot (222) and the single axis (262) of rotation is disposed on the superior arm (230).

4. The device (100) according to claim 3, wherein the other arm of the pair of arms, an inferior arm (240), is disposed inferior (I) of the slot (222) and is provided with an exit aperture (244), wherein the exit (244) aperture is an aperture passing between a superior (S) surface of the inferior arm (240) and an inferior (I) surface of the inferior arm (240) and which extends longitudinally to the proximal (10) end of the inferior (I) arm (240), and is open at its proximal (10) end.

5. The device (100) according to any one of claims 1 to 4, wherein the delivery head (200) further comprises at least one retractable locking pin (270) having a deployed and retracted position, wherein
- in the deployed position at least a part a pin shaft of the at least one retractable locking pin (270) is disposed within the slot (222), and
- in the retracted position, the slot (222) is devoid of the pin shaft.

6. The device (100) according to claim 5, wherein at least one retractable locking pin (270) is biased in the deployed position.

7. The device (100) according to claim 5 or 6, wherein the least one retractable locking pin (270) is manually actuatable between the deployed position and the retracted position using a pullcord.

8. The device (100) according to any one of claims 1 to 7, wherein the holding element (220) comprises one or more frictional contact protrusions (280) configured to increase frictional retention of the graft sheet in the slot (222).

9. The device (100) according to any one of claims 1 to 8, wherein the delivery head (200) further comprises an atraumatic distal tip (280) disposed at a distal (20) end of the delivery head (200), and the atraumatic distal tip (280) is provided with one or more surface channels disposed in a longitudinal direction configured to provide liquid drainage over an exterior of the atraumatic distal tip (280) in a distal (20) to proximal (10) direction.

10. The device (100) according to any one of claims 1 to 9, wherein the coupling element (260) comprises a coupling element body (264) having a proximal end (10) and a distal end (20), and the coupling element body (264) comprises a through passage (266) open ended at both its proximal end (10) and its distal end (20), configured for slidable coupling with and attachment to the delivery tube (310) of the tacker (300).

11. The device (100) according to any one of claims 1 to 10, wherein the coupling element (260) is further configured for slidable attachment to a flexible insertion tube of an endoscope, wherein the endoscope is configured to capture images from a distal tip of the flexible insertion tube.

12. The device (100) according to claim 11, wherein the coupling element body (264) further comprises a further through passage (269) configured for slidable coupling with the flexible insertion tube of the endoscope.

13. The device (100) according to claim any one of claims 1 to 12, wherein:
- the rotation between the coupling element (260) and the holding element (220) around the single axis (262) is realised by a revolute (1 DOF) joint (268),
- the revolute joint (268) comprises:
- one pair of revolute joint, RJ, first bodies (268a1, 268b1) disposed in fixed relation to the coupling element body (264), and
- one pair of RJ second bodies (268a2, 268b2) is disposed in fixed relation to the holding element body (220, 222),
and
- each RJ first body (268a1, 268b1) is configured to attach to the corresponding RJ second body (268a2, 268b2), thereby forming the revolute joint (268).

14. The device (100) according to claim any one of claims 1 to 13, wherein:
- the slot (222) is longitudinal, and the longitudinal length of the slot is oriented in a longitudinal direction with respect to the delivery head (200);
- the axis of rotation is oriented in a lateral direction with respect to the delivery head (200).
